(19) **Europäisches Patentamt**
**European Patent Office**
**Office européen des brevets**

(11) **EP 4 649 825 A1**

(12) **EUROPEAN PATENT APPLICATION**
published in accordance with Art. 153(4) EPC

(43) Date of publication:
**19.11.2025  Bulletin 2025/47**

(21) Application number: **23915854.6**

(22) Date of filing: **13.02.2023**

(51) International Patent Classification (IPC):
*A01N 43/16* (2006.01)     *A01N 31/08* (2006.01)
*A61K 31/722* (2006.01)    *A61K 31/192* (2006.01)
*A01P 1/00* (2006.01)

(52) Cooperative Patent Classification (CPC):
**A01N 31/08; A01N 43/16; A61K 31/192;
A61K 31/722;** A01P 1/00

(86) International application number:
**PCT/ES2023/070077**

(87) International publication number:
**WO 2024/149912 (18.07.2024 Gazette 2024/29)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **11.01.2023  ES 202330013**

(71) Applicant: **Bison Scientific SL
28042 Madrid (ES)**

(72) Inventor: **IRIARTE CELA, Mercedes
28042 MADRID (ES)**

(74) Representative: **Isern Patentes y Marcas S.L.
Avda. Diagonal, 463 Bis, 2°
08036 Barcelona (ES)**

(54) **PRODUCT WITH ANTI-OOMYCETE PROPERTIES TO CONTROL DISEASES IN SILVICULTURE, AGRICULTURE AND AQUAPONICS**

(57)     The present invention relates to a product with anti-oomycete properties to control diseases in silviculture, agriculture and aquaponics. The product comprises 1% by weight of tannic acid and 1% by weight of chitosan, which, when combined by mixing in a solution with 1% acetic acid and synthesised in a temperature range between 50 and 70°C, produce a liquid with a strong anti-oomycete effect with slow and progressive water-solubility, ensuring that the product continues to release product in aqueous solution for at least 48 hours. This product has been successfully tested in both laboratory and real environments for treating salmon affected by SAPROLEGNIA, Quercus trees and pepper plants affected by PHYTOPHTHORA, and crayfish affected by APHANOMYCES ASTACI. All this experimentation does not limit the scope of use of the product in other oomycete prevention, treatment and control applications.

EP 4 649 825 A1

**Description**

**FIELD OF THE ART**

The present invention relates to a product of a new synthesis process in the chemical field. The product is applicable for the prevention, treatment and control of the growth of oomycetes or pathogenic pseudofungi in silviculture, agriculture and aquaponics.

**BACKGROUND OF THE INVENTION**

This product is developed to solve the problem of infection caused by oomycetes transmitted through aqueous media, such as SAPROLEGNIOSIS, late blight (PHYTOPHTHORA) and the disease caused by APHANOMYCES ASTACI. This problem has not been solved, either because the products used have side effects on individuals, humans or the environment, or because they are not sufficiently effective.

Saprolegniosis is a disease caused by several pathogenic species of the genus SAPROLEGNIA that attack aquatic organisms in their embryonic stages. The economic losses caused by saprolegniosis are estimated at millions of Euros. Until 2002, this disease was kept under control in aquaculture thanks to the use of malachite green. However, the use of malachite green has been banned worldwide due to its carcinogenic and toxicological effects. This has resulted in the lack of protection for freshwater fish aquaculture.

Holm oak dieback is a disease caused by the pathogenic species PHYTOPHTHORA CINNAMOMI. Holm oak dieback is currently one of the greatest threats to the viability of Iberian woodlands and has a significant negative economic impact, especially in cork oak groves, justifying the urgent search for alternatives to develop new strategies for controlling this disease. Treatments for any PHYTOPHTHORA species are complicated and none has been able to effectively control the effects of these pathogens. Preventive treatments against the spread of P. CINNAMOMI are particularly important, as this is an important factor in the rapid mortality of oak trees in Spain and Portugal.

Aphanomycosis, or crayfish plague, is a disease caused by the pathogenic species APHANOMYCES ASTACI. Aphanomycosis is currently the greatest threat to the survival of most crayfish species worldwide, especially in Europe, which justifies the urgent search for alternatives to develop new strategies to control this disease. To date, there is no treatment for this disease, either preventive or curative.

The product presented in this invention solves the problems described thanks to its behaviour in solution, with a slow and progressive release of the active ingredients, allowing a minimum concentration to be maintained in water, particularly in open systems such as fish farms or extensive land, resulting in the inhibition of the oomycete in the transmission and contagion medium.

The state of the art with respect to similar products containing analogous compounds developed in other states of matter and for purposes other than those described in this invention is described in detail below:
Document Rubentheren et al. 2015 describes the preparation of liquid compositions containing chitosan and tannic acid. The compositions are prepared by dissolving chitosan (2%) in an acetic acid solution (2%) and stirring for 3 hours at a temperature of 35°C, and mixing this solution with tannic acid (20 or 40 mg of tannic acid/g of chitosan) while continuing to stir and maintaining the temperature for another hour. These compositions are used to produce films by evaporation on a surface (sections 2.3 and 2.4, Table 1, samples II A and B).

Document CN105079878A describes a composition including chitosan, acetic acid and tannic acid which is used to produce an antibacterial coating. The process for obtaining the coating comprises the step of mixing, with stirring, a solution of chitosan dissolved in acetic acid (1%) with a tannic acid solution, and then depositing the mixture on a surface (see examples). The composition used for the coating has the same main ingredients (chitosan, acetic acid and tannic acid) and in the same proportions as the composition of the invention (1 to 4% chitosan and tannic acid).

The documents Rivero et al., 2010 and Rivero et al., 2011 disclose the preparation of liquid compositions comprising solubilising chitosan (1.5%) in an acetic acid solution (1.5%) and mixing, with stirring, with different proportions of tannic acid (20-80 mg/g of chitosan) to produce films.

Document US2011059162A1 discloses various compositions containing chitosan and tannins. Tannins are added to the formulation in order to improve the antibacterial and antifungal properties and/or the mechanical properties of compositions that only include chitosan (paragraphs 3-5). The patent demonstrates that bacteriostatic and fungistatic activities increased when both compounds were used (paragraphs 151 and 152, Table 12). The tannins used in the composition may be condensed and hydrolysable, preferably non-monomeric, although the composition may contain up to 5% monomeric tannins such as tannic acid (paragraphs 6, 59).

Document EP3318125A1 discloses a composition for use in the treatment of diseases caused by fungi and other plant pathogens containing chitosan, an aqueous extract of Castanea sativa rich in tannins and citric acid (Example 3).

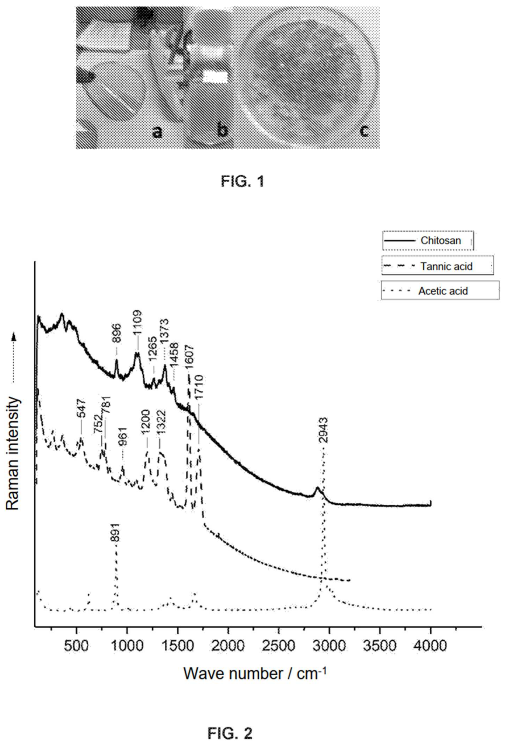

Document Lemke et al. 2022 shows the antifungal activity of chitosan dissolved in acetic acid and mixtures of chitosan and copper against a fungus of the genus Fusarium (Figure 2, Table 2)

Document paper Huang et al. 2021 is an article on the activity of chitosan solutions in acetic acid against the oomycete PHYTOPHTHORA infestans. The growth of the pathogen was completely inhibited at the highest concentration of chitosan used, with the degree of inhibition depending on the concentration of chitosan included in the composition. The composition also affected the germination of oomycete spores (sections 3.1 and 3.2).

Document ES2761076A1 discloses compositions for eliminating pathogenic agents for agriculture, such as fungi (Fusarium culmorum) and pseudofungi (PHYTOPHTHORA cinnamomi). Said compositions (conjugated compounds) comprise chitosan oligomers and antimicrobial compounds, such as polyphenolic acids. To obtain the chitosan oligomers, the chitosan is first dissolved in acetic acid with stirring at a temperature of 60°C (page 5, line 29-page 8, line 18; page 11, lines 5-15, tests 1 and 2).

Document Min et al. 1994 shows the inhibitory effect of chitosan on the pathogenic oomycete SAPROLEGNIA parasitica in fish (see Figures 1 and 2).

Document Stössel and Leuba 1984 refers to the use of chitosan solutions with acetic acid at different pH values to inhibit the growth of oomycetes of the genera APHANOMYCES and PHYTOPHTHORA and other phytopathogens. At pH 4, none of the oomycetes grow (results, Tables 2 and 3).

Document Ahmed et al. 2020 is a study of the activity of chitosan (in suspension or in nanoparticles) against various pathogenic bacteria and oomycetes in fish. Chitosan nanoparticles were effective against many of the bacteria and oomycetes (section 3.3). The suspensions showed no effect against the pathogens because they were used at a pH higher than the suitable pH, according to the authors (section 4, discussion).

Document Zhang et al. 2019 studies the effect of tannic acid solutions at various concentrations on the pathogenic oomycete SAPROLEGNIA parasitica in aquaculture (Figure 1, Table 1).

## DESCRIPTION OF THE INVENTION

The present invention relates to a product obtained by a new synthesis process (referred to as BioCopal in the supplementary reports of the Spanish National Research Council (CSIC) where the microbiological analyses were carried out), with physicochemical properties that give it a prolonged anti-oomycete effect. The product is a liquid denser than water, which diffuses slowly in aqueous solution for up to at least 48 hours and acts against the growth of pseudofungi.

Method of production

Reagents:

a) Ethanoic acid ($CH_3COOH$)
b) Chitosan (any deacetylation degree)
c) Tannic acid (2,3-dihydroxy-5-({[(2R,3R,4S,5R,6R)-3,4,5,6-tetrakis({3,4-dihydroxy-5-[(3,4,5-trihydroxyphenyl)carbonyloxy]phenyl}carbonyloxy)oxan- 2-yl]methoxy}carbonyl)phenyl 3,4,5-trihydroxybenzoate)
d) Additives and colouring agents

A 1% acetic acid solution by volume is prepared. A given volume of this solution is heated in the range 50 to 70°C, preferably in a bain-marie. Once heated, 1% by weight of chitosan with respect to the volume of the solution is added, with constant stirring. Once the chitosan is dissolved, 1% by weight of tannic acid with respect to the total volume is added in the same solution. The mixture is stirred vigorously, always maintaining the temperature, for the time necessary for complete dissolution (about 1 hour). Once dissolved, a percentage by weight/volume of not more than 5% of any authorised additive or colouring agent may be added. It is left to cool at room temperature and packaged in bottles protected from sunlight and at temperatures below 20°C. The final product is a viscous liquid with an orange-brown colour and a pH of around 4.

Physicochemical analyses

Experimental tests, both in the laboratory and in relevant environments, relating to each of the diseases described in the introduction have been carried out by applying the product in different states of matter using the same formulation, Figure 1, or formulations found in other publications or patents with similar compounds but with different methods of production. Only this product in liquid state, synthesised at a temperature between 50 and 70°C, offers the advantages of effectiveness as an anti-oomycete in the media studied. To verify these advantages, tests were carried out with the different products:

a) Liquid. The product is tested with two types of synthesis:

a. Synthesis at room temperature.

b. Synthesis at a temperature between 50 and 70°C.

b) Liquids described in Rubentheren et al. 2015.

a. Rubentheren et al. 2015 with 0.004% tannic acid.
b. Rubentheren et al. 2015 with 0.008% tannic acid.

c) Solid. The product can be solidified by natural curing at room temperature on a surface, or by accelerated curing by means of an oven at temperatures between 55 and 120°C, resulting in a biofilm. It is also possible to generate films by electrophoretic deposition on metal surfaces.

d) Gel. Gelation can be generated by means of ionic gelation, by immersing a portion of the product in a solution with a concentration of 1M or higher of NaOH, or by absorbing the liquid product in superabsorbent inert gels, such as potassium polyacrylate.

In no case is the product presented as a nano-coating. None of the methodologies described in document CN105079878A, such as electrolysis in coordination with electrophoretic deposition, the function of which is to generate a nano-sized coating, are used in the production of this product. The methodology described in patent US2011059162A1 is not used either, as it does not involve nanoparticles, hydrogel film, biofoam, biogel or as a solid or liposome coating. On the contrary, the present invention does not seek a coating or a support for drug product release. The effectiveness of the liquid solution as a fungicide in aqueous solution has been verified in comparison with presentations of the product in solid forms, such as a coating film or gel. Chitosan, which is the basis of the product, is obtained from crustaceans and is a biopolymer of non-toxic, readily absorbed amino sugars. Its properties as a fungicide are already known, and it is applied in bandages for its anti-haemorrhagic and antimicrobial properties[1] Rafaat, Dina et al. Microbial Biotechnology 2 (2009) 186-201.. However, these properties are not powerful enough to kill pathogenic OOMYCETES, so the formulation of this invention greatly improves its anti-oomycete effect without harming the ecological or natural environment.

The product, as will be demonstrated below, offers the following advantages over other products synthesised without temperature or those described in the patents cited or in the publications listed in the background of the invention:

a) The production method is easy and inexpensive. No electrophoresis equipment, nanoparticle or micelle generation, or curing processes are required.

b) The application of temperature in the synthesis process is vital to generate a very homogeneous product with a modified polymer matrix that allows optimal behaviour in aqueous solution against pathogenic oomycetes.

c) The product has a much stronger anti-oomycete effect than other products, all tested in Petri dishes.

d) The release of the components in aqueous solution is slow and progressive over at least 48 hours only for the product described in this specification, which is not the case with other products. This particularity gives it a powerful long-term effect in aqueous media, inhibiting the growth of pathogenic pseudofungi with 40% greater effectiveness than products with a synthesis methodology below the temperature of the product herein presented, 80% more effective than gel-type products, and 100% more effective than solid products.

e) The slow release of the product components in aqueous solution is a very important technical advantage, as it prevents overdosing of the medium to be controlled and overexposure of the species (animals or plants) to a continuous addition of the product to maintain a constant concentration of the product, inhibiting the growth of the oomycete,

f) The product is shown to be harmless and non-toxic to fish, plants, and crayfish.

g) Chitosan serves not only as a support matrix, but also has its own anti-oomycete effect and, in the case of plant species, boosts their natural immune system[2] Zheng, K. et al. International Journal of Biological Macromolecules 182 (2021) 1670-1680..

Solid and liquid products are analysed using Raman and UV-Visible spectroscopy. Ionic gelation results in a rubbery compound as the final product that loses all tannic acid, completely changing the product's effectiveness and rendering it inert to pathogens. The gel in potassium polyacrylate has the same composition as the liquid, which is why only the solid and liquid products are taken into account for analysis.

Raman spectroscopy

The characterisation of the products is analysed by means of Raman spectroscopy, using Renishaw InVia equipment with two laser lines at 532 and 785 nm, with exposure times between 10 and 20 seconds and a maximum of 32 accumulations.

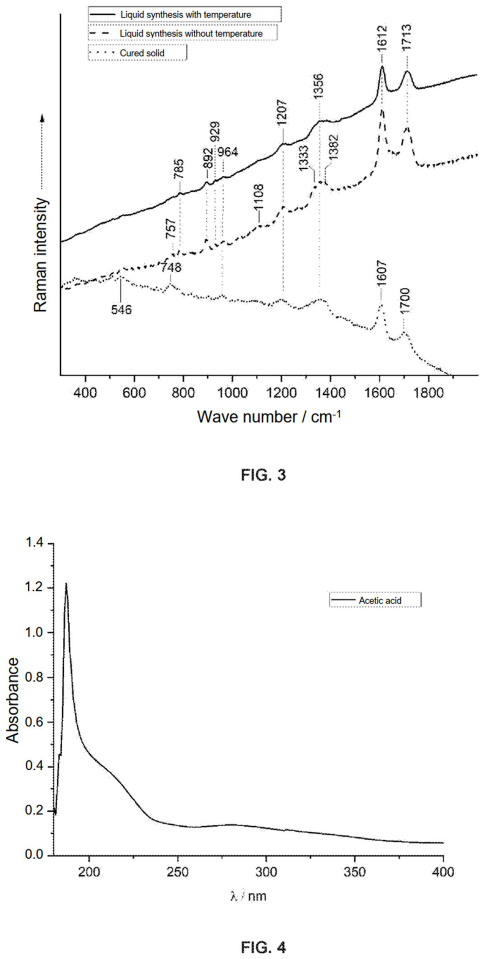

Figures 2 and 3 show the Raman spectra of the pure compounds used in the formulation, and the Raman spectra of the product for the different liquid and solid syntheses, respectively. In the Raman spectra of the original product, the bands between 1607 and 1713 cm$^{-1}$ correspond to tannic acid, maintaining the intensity ratio and spectral profile for the liquid, but not for the solid, where they have a greater mean band width and lower intensity. This peculiarity reveals that the compound, even when present, is much more affected in the vibration modes due to the chemical environment of the polymer. On the other hand, the bands in the spectral range between 540 and 1400 cm$^{-1}$ correspond to chitosan and tannic acid bands. These bands increase in mean width and lose the defined profile of the bands in the case of the solid product in film form, Figure 3 dotted line, denoting a loss of crystallinity in its atomic-molecular structure[3] Stuart, B.H. Vibrational Spectroscopy 10 (1996) 79-87. Available at: https://www.sciencedirect.com/science/article/pii/0924203195000429. This is particularly well observed in the area between 1300 and 1400 cm$^{-1}$, where the symmetrical vibration bands of tension and deformation in the plane of the -CH$_2$, -CH, -OH and -CN groups[4] Zajac, A. et al. Spectrochimica Acta Part A: Molecular and Biomolecular Spectroscopy 134 (2015) 114-120. Available at: https://www.sciencedirect.com/science/article/pii/S1386142514009597 disappear with respect to the spectra of the liquid product and the original chitosan. Figure 3.

In the case of the liquid product, two different syntheses are studied, with and without temperature, observing that, although the product synthesised at room temperature retains well-defined bands between 700 and 1400 cm$^{-1}$, the liquid synthesised with temperature retains the bands, although they are less intense and narrower. This physicochemical evidence shows a structural difference between the solid-cured product and the liquid synthesised with and without

temperature. The solid film is shown as an amorphous solid where only the tannic acid bands are clearly distinguishable. On the other hand, the effect of temperature on liquid synthesis favours the inclusion of tannic acid molecules in the polymer structure due to the electrostatic affinity of the functional groups of both compounds, such as aryl groups, rich in electrons, amino and hydroxyl groups, and this inclusion slightly undermines the original crystalline structure of chitosan without breaking it (otherwise the Raman spectrum would show other more pronounced differences). Temperature control allows the structure of chitosan to be maintained while preserving the structure of tannic acid, both perfectly integrated into a chitosan-tannic acid molecular structure, generating a liquid compound that is slightly more viscous and denser than water, with the particularities of tannic acid and chitosan release and diffusion into the surrounding medium described below, which particularities are not present in the compound synthesised without temperature control, nor are they found in other states of matter such as solid films, solid coatings, biogels or biofoams.

UV-Visible Spectroscopy

Another very important difference that justifies the use of the product exclusively in the form of a liquid synthesised with a specific temperature is its behaviour in aqueous solution. NanoDrop ONE UV-Vis and Shimadzu UV-Vis UV-3600i plus equipment are used. Measurements up to 70 minutes are taken with the latter, as it is much more sensitive.

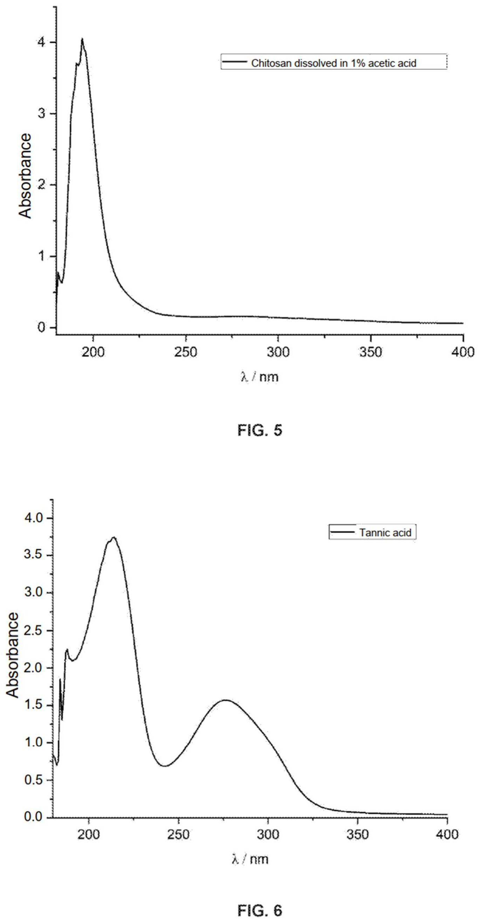

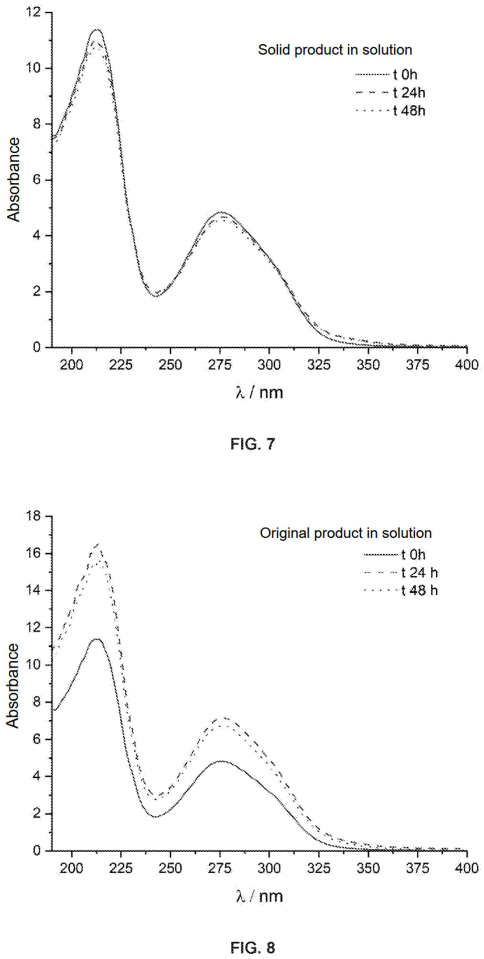

The pure reactants are measured separately by UV-Vis, Figures 4-6. By taking solid sheets of the bioproduct and immersing them in water, as well as the equivalent amount of the liquid product synthesised with temperature immersed in water, a completely different behaviour over a period of 48 hours can be verified by means of UV-Visible spectroscopy. This test was decisive in enabling the product to be administered in real-world environments, both in fish farms with salmonids and in tests with crayfish, as well as in irrigation water for plants. While the solid releases all the tannic acid immediately and the concentration is stabilised from the outset, Figure 7, the liquid product shows a progressive release over time, Figure 8. Also note that the intensity of the absorbance, which is directly related to the concentration of the product in solution according to Lambert-Beer's equation, is much lower for the release of the compound in the solid, at a maximum of about 12 units, while in the case of the liquid product, where it begins to release concentrations of 12 units at time 0, after 48 hours it continues to release equivalent concentrations above 16 units.

Likewise, the test is carried out at dissolution times of 70 minutes in order to better detail its behaviour, comparing the following compounds:

    a) Liquid.

        a. Synthesis product at room temperature (23°C).
        b. Synthesis product at 35°C.
        c. Synthesis product at a temperature between 50 and 70°C. This one in particular was synthesised with a mean temperature of 60°C, and it never dropped below 55°C.

    b) Liquids described in Rubentheren et al. 2015.

a. <u>Rubentheren et al. 2015</u> with 0.004% tannic acid.
b. <u>Rubentheren et al. 2015</u> with 0.008% tannic acid.

<u>Experimental procedure</u>

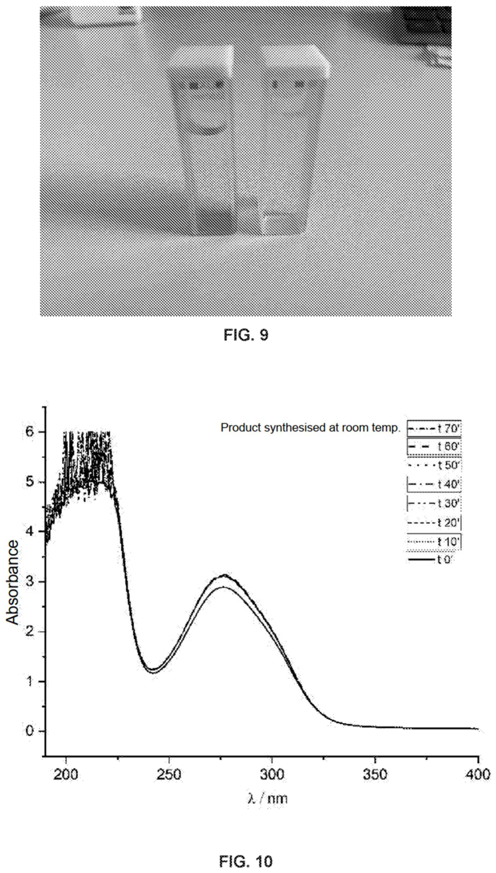

To perform UV-Vis analyses, a quartz cuvette is used, into which 3 ml of type III ultrapure water is placed, Figure 10. For each case, 20 μl of the product are added and the UV-Vis spectra are measured every 10 minutes until a total of 70 minutes has elapsed.

<u>Results</u>

As can be seen in Figure 9, the product synthesised with a temperature between 50 and 70°C is initially more insoluble and settles at the bottom, while the product synthesised at room temperature (23°C) and the product synthesised at 35°C dissolve immediately. The results of the latter two products are virtually identical.

*Synthesis product at room temperature (23°C)*

The result of the test shows that as soon as the product is added to the water, it dissolves almost completely, Figure 10, and after 10 minutes, it is completely dissolved, with no spectroscopic changes observed over time. The vertical lines below 250 nm appear due to the saturation of the instrumental equipment in that spectral range.

*Synthesis product at 35°C*

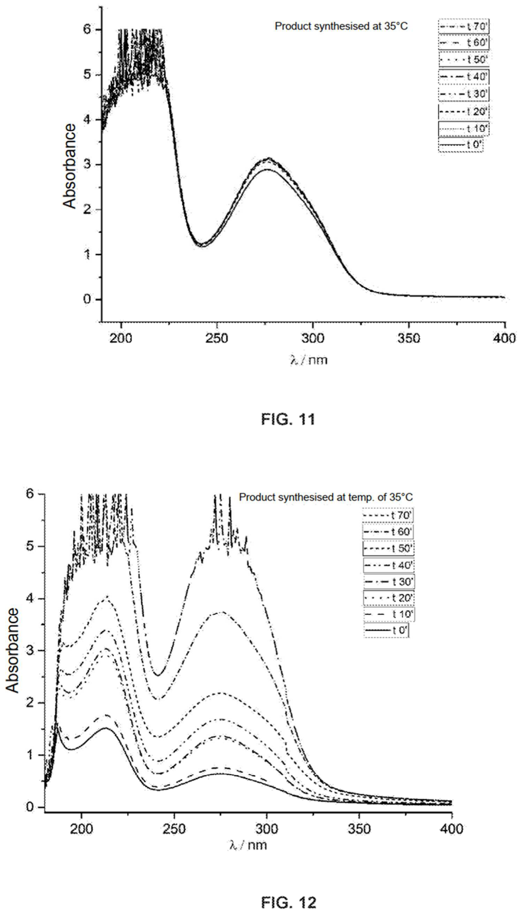

The UV-Vis spectrum shown in Figure 11 is virtually identical to that of the product synthesised at 23°C. Only one of the spectra, the one measured at 20 minutes, appears slightly below the following spectral measurements. That is, the behaviour is similar to that of the product synthesised at 23°C.

*Synthesis of the product at a temperature of 50 to 70°C*

This product has a very different behaviour in solution compared to that shown by the other products. As shown in Figure 12, the release of tannic acid and chitosan is slow and progressive over time, which is a very important quality for the treatment of diseases in aqueous media, as it prevents overdosing of the medium to be controlled and overexposure of the species (animals or plants) to a continuous addition of the product. After 70 minutes, the concentration in water has saturated the UV-Vis equipment detector. Although initially the amount of product measured at time 0 in water is less than the equivalence of 1 absorbance unit, taking the maximum band at 279 nm as a reference, after a period of time, the concentration of the product increases above 5 absorbance units, whereas in the product at room temperature, concentrations above 2 absorbance units are released from the beginning and never exceed 3.2 units, with no changes observed over time.

*<u>Rubentheren et al. 2015</u> with 0.004% tannic acid*

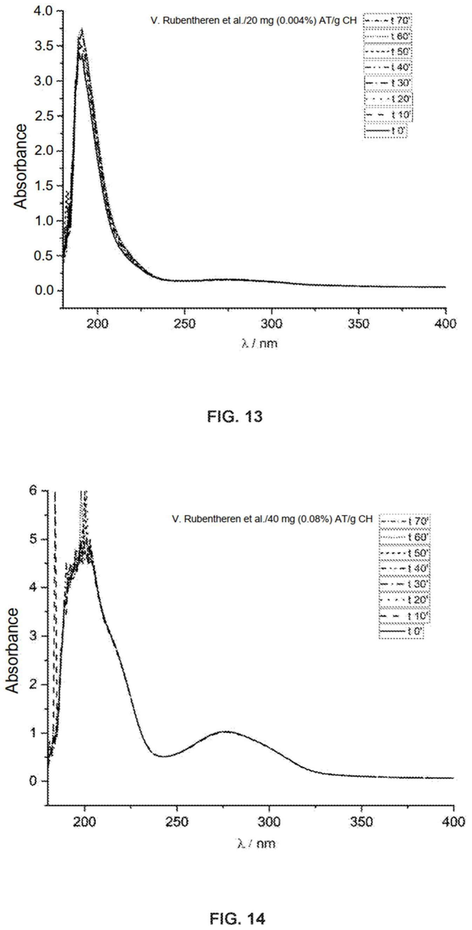

Figure 13 shows the behaviour of the product synthesised following the indications described in the article referred to, with a chitosan concentration of 2%, an acetic acid concentration of 2%, and a tannic acid concentration of 0.004%. There is no progressive dilution in any case, and the tannic acid concentration is very low and is released immediately.

*<u>Rubentheren et al. 2015</u> with 0.008% tannic acid*

In the case of the product described in the aforementioned article containing a chitosan concentration of 2%, an acetic acid concentration of 2%, and a tannic acid concentration of 0.008%, the behaviour is similar to that of the previous section, as shown in Figure 14. The band at 279 nm becomes more intense as the amount of tannic acid is higher; however, the release of the compounds is immediate.

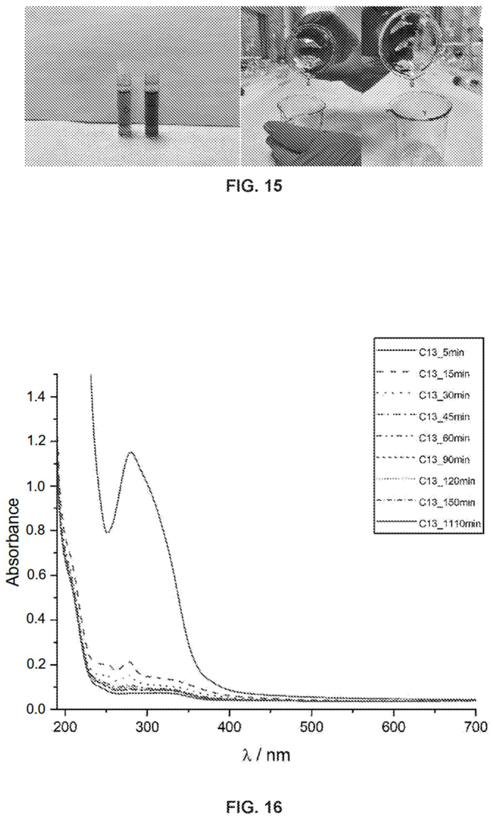

In addition to the difference in structure and dilution of the original product compared to the other products described in other patents or publications, there is another difference between the liquid product synthesised at room temperature and that synthesised with heating when photochemical ageing is induced in both products. When the two products are subjected to continuous exposure to sunlight, changes in colour and texture are observed. Thus, it can be observed that the product synthesised at room temperature darkens much less and remains a light liquid; on the other hand, the product synthesised with temperature control takes on a much darker colour and becomes a much thicker and denser liquid, Figure 15.

The temperature therefore favours the loss of structural independence of the chitosan polymer, as demonstrated in the Raman analysis, due to the inclusion of tannic acid in the polymer matrix, resulting in a very homogeneous and well-distributed liquid as the final product. This relationship between the two molecules shows more visible effects under photochemical ageing because tannic acid is very sensitive to radiation, changing colour (due to electron changes in the functional groups), which also affect the chitosan matrix, changing the colour of the entire product. Meanwhile, without temperature, chitosan remains stable under ageing, probably due to the protection provided by a micellar structure around the tannic acid, keeping the two compounds separate and ensuring that the product does not discolour. Therefore, it is known that the product must be stored and preserved in dark conditions. Synthesis with temperature must be carried out under the conditions described and never above 90°C, as the polymer structure breaks down, generating random polymer structures with a rubbery appearance and losing their antimicrobial effectiveness.

Results of laboratory tests of the product on PHYTOPHTHORA, SAPROLEGNIA and APHANOMYCES ASTACI microorganisms

Laboratory tests on Petri dishes with cultures of SAPROLEGNIA, PHYTOPHTHORA and APHANOMYCES yielded very revealing results. The difference between the solid and liquid products on the growth of these pathogens in Petri dishes is pivotal for their use and application. As can be seen in Table 1, the inhibition halo against SAPROLEGNIA is clearly larger when a 5 $\mu$l drop of the original product is used, whereas when a coating film or biogel is used, its effectiveness is significantly lower. The same results are obtained with other pathogens.

The physicochemical difference described in the previous section for the different presentations of the product in its molecular structure, between the solid product and the liquid product, justifies the results of the tests in Petri dishes. Dr Javier Diéguez Uribeondo, a scientific researcher at CSIC, was responsible for testing the product on microorganisms. Laboratory tests with Petri dishes show that the presence of the product causes the encystment and lysis of the zoospore and, therefore, prevents the mobility of the zoospores and prevents germination, thus preventing contagion. These effects prevent dispersion in the medium and, therefore, would prevent the spread of these pathogens in different media.

Table 1 shows the results of the test with all the products, reagents and synthesis alternatives. Solid film samples, gels and liquid samples were used. The best results are seen for the sample of the original product, with inhibition halos >8 mm in radius in the Petri dish, followed by samples of the product synthesised at room temperature (23°C) and at 35°C. In gel, they show much smaller inhibition halos, 5 or 1 mm in radius, whereas the solid film samples had no effect. In the expert's opinion, small halos, >2-3 mm, may be carry-over halos, which is why in the case of dry gel and the product of Rubentheren et al. 2015 with 0.008% tannic acid, there is no inhibition of oomycete growth.

Taking into account the effects described above, the **original product is about 40% more efficient in a Petri dish than the liquid product synthesised at room temperature or at a temperature of 35°C, 80% more efficient than the gel application, and 100% more efficient than the solid film application.**

**Table 1.** Summary of results two weeks after contact between different products and SAPROLEGNIA zoospore suspension (results are similar to those obtained with PHYTOPHTHORA and APHANOMYCES ASTACI), and description of the physical behaviour of each product in aqueous solution.

| Product | Photograph | Halo/r* (mm) | Inhibition of oomycete growth within the halo** | Behaviour of the product in aqueous solution |
|---|---|---|---|---|
| **Original (synthesis in a temperature range between 50 and 70°C)** | | Yes >8 mm | Yes | Dilutes slowly, in a process of progressive diffusion over time of at least up to 48 hours |
| **Synthesis at temperature 23°C** | | Yes 4-5 mm | Yes | Dilutes almost completely and immediately |
| **Synthesis at temperature 35°C** | | Yes 4-5 mm | Yes | Dilutes almost completely and immediately |

(continued)

| Product | Photograph | Halo/r* (mm) | Inhibition of oomycete growth within the halo** | Behaviour of the product in aqueous solution |
|---|---|---|---|---|
| **Air-cured solid film** | | No | No | Dilutes immediately, releasing less than half the concentration of the compounds than in liquid products |
| **Oven-cured solid film** | | No | No | Dilutes immediately, releasing less than half the concentration of the compounds than in liquid products |
| **Potassium polyacrylate gel with original product** | | Yes 5mm | Yes | Dilutes at a very low concentration and immediately |
| **Dried potassium polyacrylate gel with original product** | | Yes 1 mm | No | Does not dilute and takes water intake from the environment |
| **Ionic gel** | | Yes 1 mm | No | Does not dilute |
| **1% tannic acid** | | Yes 3-4 mm | Yes | Dilutes immediately |
| **Chitosan diluted to 1% in a 1% acetic acid solution** | | No | No | Dilutes immediately |
| **Rubentheren et al. 2015 with 0.004% tannic acid** | | No | No | Dilutes immediately |
| **Rubentheren et al. 2015 with 0.008% tannic acid** | | Yes 1 mm | No | Dilutes immediately |
| *r: halo radius quantified from the product boundary.<br>** Tests are performed in a PGA agar Petri dish. | | | | |

## BRIEF DESCRIPTION OF THE DRAWINGS

To complement the description being made and to aid in a better understanding of the features of the invention, a set of figures is attached as an integral part of said description, in which, by way of illustration and not limitation, the following is depicted:

Figure 1. The product shows the same composition, but with a different synthesis process: a, on solid film; b, liquid solution of the original product, and c, gel.

Figure 2. Raman spectra of the pure compounds separately, i.e., chitosan, tannic acid and acetic acid.

Figure 3. Raman spectra of the original product, of the liquid product with synthesis at room temperature, and of the cured solid.

Figure 4. UV-Vis spectrum of 1.6 10⁻⁴ M acetic acid in aqueous solution.

Figure 5. UV-Vis spectrum of 0.01% chitosan (w/v) in acidified aqueous solution.

Figure 6. UV-Vis spectrum of 0.01% tannic acid (w/v) in aqueous solution.

Figure 7. UV-Vis spectrum of the solid product in solution at zero time, at 24 hours, and at 48 hours.

Figure 8. UV-Vis spectrum of the original liquid product (temperature between 50 and 70°C), in solution at time zero, at 24 h, and at 48 hours.

Figure 9. Image of the quartz cuvettes with the original product on the left, and with the synthesised product at 35°C on the right.

Figure 10. UV-Vis spectra of the product synthesised at room temperature (24°C) measured every 10 minutes in aqueous solution.

Figure 11. UV-Vis spectra of the product synthesised at 35°C measured every 10 minutes in aqueous solution.

Figure 12. UV-Vis spectra of the original product, synthesised in the range comprised between 50 and 70°C, measured every 10 minutes in aqueous solution.

Figure 13. UV-Vis spectrum of the product described in Rubentheren et al. 2015 with 0.004% tannic acid, measured every 10 minutes in aqueous solution.

Figure 14. UV-Vis spectrum of the product described in Rubentheren et al. 2015 with 0.008% tannic acid, measured every 10 minutes in aqueous solution.

Figure 15. Images of the photoaged products synthesised with and without temperature. From left to right, vials with the product synthesised without temperature and with temperature; drop of the product synthesised without temperature, very liquid; drop of the product synthesised with temperature, thick and dense.

Figure 16. UV-Vis spectra of the evolution of the concentration of the product over time inside the trough.

Figure 17. A representation of the evolution of the product concentration over time and its corresponding mathematical function.

Figure 18. UV-Vis spectra of the product solutions in water in the fish farm test.

Figure 19. The least squares fit of the data taken with UV-Vis at 282, 282.5, 283, and 283.5 nm.

Figure 20. Two photographs of eggs treated with the product (left) and untreated eggs (right).

Figure 21. Photographs of the hatching period of the eggs. Left, unhatched eggs infected with SAPROLEGNIA from the control; right, hatched healthy fry from the treated trough.

## PREFERRED EMBODIMENT OF THE INVENTION

As detailed in the description of the formulation, the synthesis of the product is readily susceptible to being industrialised and does not present any problems for scaling up, as has been verified in laboratory tests, from 100 ml to production in a 5L industrial reactor, obtaining the same final product. Three experimental tests in real-world environments for each application are described below by way of example and are not intended to be limiting of their scope.

Example 1

APPLICATION TESTS FOR THE PRODUCT AGAINST SAPROLEGNIOSIS IN SALMON AT THE SAN FRANCISCO DE ASIS FISH FARM, ORONOZ- MUGAIRE, NAVARRE.

SUMMARY

The product is tested in a real-world environment at the San Francisco de Asís fish farm in Oronoz-Mugaire, Navarre. An experiment is designed for the curative and preventive treatment of *saprolegniosis* in salmon eggs and adult salmon. The methodology consists of dissolving the bioproduct in the troughs with the fertilised salmon eggs and applying the product topically and in solution to the diseased salmon. The results obtained show a clear preventive antifungal activity on salmon eggs, inhibiting growth of the fungus and with a hatching success rate of **100%,** with **0% infected and with live and healthy fry.** Meanwhile, in the control (untreated) eggs, only 75% of the eggs hatch, 25% are infected, did not hatch and died. The use of the product on adult salmon prevented transmission of the disease.

INTRODUCTION

The proliferation of SAPROLEGNIA in fish species on fish farms has been extensively studied[5] Sandoval-Sierra, J.V., et al. Aquaculture, 2014; vol. 434, 462-469. [6] Hendrick Van den Berg, A., et al. Fungal Biology Reviews, 2013; vol. 27-2, 33-42. The need to find a bioproduct that is non-toxic to animals and does not pollute the environment has proved to be a challenge in recent years. A polymer of natural origin with fungicidal properties that is environmentally friendly and safe for the animal species to be treated, specifically salmon, has been synthesised.

The Government of Navarre has authorised a test in a real-world environment at the *San Francisco de Asis* fish farm in Oronoz-Mugaire (Navarre) in February, March and April 2022 for the treatment of saprolegniosis in infected adult salmon and in fertilised salmon eggs.

APPLICATION METHODOLOGY AT THE FISH FARM

Treatment at the fish farm is applied using two different methodologies: a topical treatment for salmon with SAPROLEGNIA ulcerations, and the addition of a bioproduct to the 1,000-litre tank in which they are housed; and another treatment with a bioproduct in solution in the tanks on the trestles to prevent the fungus from spreading to fertilised salmon eggs.

*Adult salmon*

Adult salmon affected by SAPROLEGNIA with very advanced ulcerations in the tail area are

treated topically by applying the bioproduct with a brush. In addition, a healthy individual is placed in the same tank to check the effect of the product on the spread of the disease. Every day, the individual is removed from the tank and anaesthetised before proceeding with topical treatment. Every three days, the product is added to the tank water for a 40-minute bath, after which the normal water cycle is resumed.

*Salmon roe*

Salmon roe is only treated with the product in solution every two days, without applying a bath, with the usual circulating water. The product is added to the bottom of the trestle and allowed to diffuse and dissolve following its natural course towards its outlet. The water enters the trestle through a grate at the bottom of same where the eggs rest. From there, the water flows out through the top part of the opposite wall of the trestle from its inlet. Along this path, the dissolved product passes through the salmon roe.

Two troughs are prepared with 20 healthy fertilised eggs and 4 infected eggs in each trough. One will be the control trough, where no product is applied, and the other will be the trough where the product is added.

The product was applied in the trough itself, because it is where the eggs are housed and where the water flows at the lowest speed. Preferably near the eggs and at the beginning of the current, so that it spreads throughout the trough.

On the first day of application of the bioproduct, samples are taken at specific times in the area inside the trough near the flow outlet to monitor the concentration of the product that is about to leave the area of action. The analyses are carried out in the laboratory with Shimadzu 3600i UV-Vis-NIR equipment.

*Water sampling results*

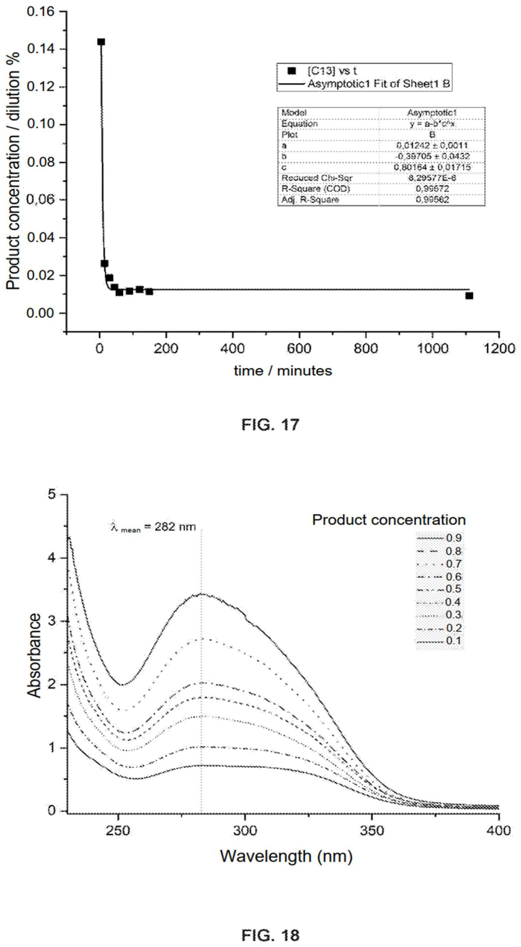

The results of the analyses show that the product dilutes rapidly at first, and its concentration decreases following an asymptotic function, Figures 16 and 17. From that point on, there is a slower release into the water. Concentrations are calculated by UV-Visible spectroscopy in relation to the maximum band at 279 nm, the calibration line of which was previously established in the laboratory, as shown below.

Experimental procedure

UV-Vis spectra of polymer solutions at different concentrations in fish farm water have been recorded.
Concentrations:

Concentrations have been calculated as a percentage (%). These are summarised in table 2 with their corresponding volumes.

**Table 2.** Volume of product corresponding to different concentrations

| VT | | 4 | ml |
|---|---|---|---|
| C (%) | $V_{Cop}$ (ml) | $V_{Cop}$ (µl) | $V_{H2O}$ (ml) |
| 0.90 | 0.036 | 36 | 3.964 |
| 0.80 | 0.032 | 32 | 3.968 |
| 0.70 | 0.028 | 28 | 3.972 |
| 0.60 | 0.024 | 24 | 3.976 |
| 0.50 | 0.020 | 20 | 3.980 |
| 0.40 | 0.016 | 16 | 3.984 |
| 0.30 | 0.012 | 12 | 3.988 |
| 0.20 | 0.008 | 8 | 3.992 |
| 0.10 | 0.004 | 4 | 3.996 |

Experimental conditions: spectral range of 175 to 600 nm, resolution of 0.5 nm, slit of 0.5 nm (fixed) and mean speed.
Spectroscopic characterisation:

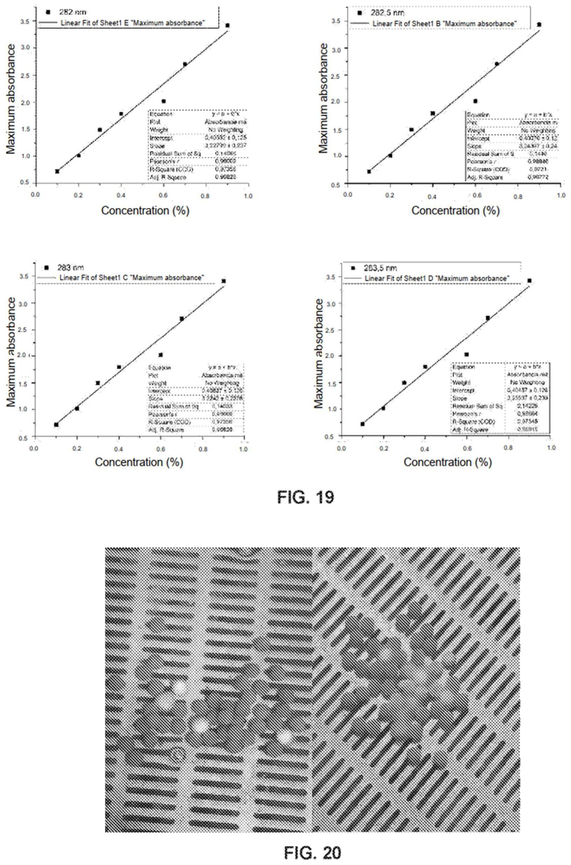

Least squares fit and calibration curve. Since the absorbance maximum is mostly obtained between 282 and 283.5 nm, the wavelength that best fits the linear equation has been evaluated. This is done using least squares fit, Figures 18 and 19.
Table 3 summarises the main parameters obtained from the least squares fit:

**Table 3.** Main parameters of the least squares fit.

| Wavelength ($\lambda$, nm) | Slope (a$\pm\Delta$a, 1/%) | Y-intercept (b $\pm\Delta$b) | Pearson's coefficient (r) | $r^2$ | Adjusted $r^2$ |
|---|---|---|---|---|---|
| 282.0 | 3.22799$\pm$0.237 | 0.40392$\pm$0.125 | 0.98669 | 0.97356 | 0.96828 |
| 282.5 | 3.24397$\pm$0.24 | 0.40076$\pm$0.12 | 0.98656 | 0.97310 | 0.96772 |
| 283.0 | 3.2242$\pm$0.2376 | 0.40837$\pm$0.125 | 0.98669 | 0.97356 | 0.96828 |
| 283.5 | 3.23937$\pm$0.239 | 0.40457$\pm$0.126 | 0.98664 | 0.97345 | 0.96815 |

The best fit corresponds to wavelengths of 282 and 283 nm. Therefore, the equation of the curve corresponds to:

$$y = ax + b\,[1] \rightarrow y = (a \pm \Delta a) \cdot x + (b + \Delta b)\,[2]$$

$$\textit{Max. abs.} = (3.228 \pm 0.237) \cdot C\,(\%) + (0.404 \pm 0.125)\,[3]$$

This equation is used to calculate concentrations in samples taken at the fish farm.
It should be noted that the water in the trough containing the eggs is not static and flows continuously, so if the product did not diffuse slowly, all of it would disappear within a few minutes.
The analysis of concentration as a function of time reveals two important aspects to consider: there is an initial rapid dilution of the product and a concentration that declines progressively but slowly after the first 15 minutes. This initial rapid dilution is attributable to the release of the tannic acid least included in the polymer matrix, as seen in the UV-Vis spectra, while the slow release of the rest of the compound corresponds to the chitosan and tannic acid that are included in the matrix itself.

*Thirteen days after the first application*

Thirteen days after the first application, a difference was observed between the trough with treated eggs and the untreated trough. In the first case, the eggs were healthy and the fungus had not spread from the diseased eggs to the healthy eggs; in the second case, the untreated eggs showed fungal growth in more than a third of them, Figure 20.

*Hatching period*

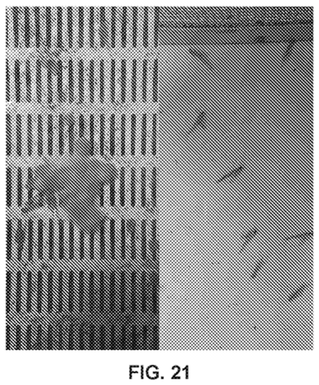

The final result of the test is a complete success. In the control (untreated) eggs, 75% of the eggs hatch and 25% are infected, do not hatch and die. **The eggs treated with the product hatch 100%, with 0% infected and with live and healthy fry,** Figure 21.

CONCLUSIONS

The test in a real-world environment at the Oronoz-Mugaire fish farm in Navarre confirmed the product's effectiveness in preventing saprolegniosis, with 100% of the treated salmon eggs hatching free of saprolegniosis, compared to untreated salmon eggs, of which only 75% hatched, with 25% being infected and dead. As a result of the 10-day treatment, the ulcerations of the adult salmon did not progress and the healthy adult individuals in the same tank did not become infected with the disease.

Example 2

APPLICATION TESTS FOR THE PRODUCT AGAINST PHYTOPHTHORA ON QUERCUS AND PEPPER PLANTS AT THE ROYAL BOTANICAL GARDEN (CSIC). MADRID

SUMMARY

The product is laboratory tested in repeated tests with strains of PHYTOPHTHORA CINNAMOMI and PHYTOPHTHORA INFESTANS with different derivatives of the product, in solid state, in gel state, and in liquid state. As in the case of SAPROLEGNIA, the liquid bioproduct is much more effective, with inhibition halos of more than 80% effectiveness compared to solid films and gel. Once the laboratory tests have been completed, an experiment is designed with cork oak and pepper seedlings in which the liquid bioproduct is being applied in order to test its effectiveness against the pseudofungus.

INTRODUCTION

Infection caused by the pseudofungus PHYTOPHTHORA in Quercus and Solanaceae such as holm oak, cork oak, pepper and tomato plants has become widespread in recent years. The treatment or prevention of this disease is proving challenging due to the difficulty of finding a product that is non-polluting, non-toxic to the tree/plant environment and easy to apply. In particular, the Iberian pig market is one of the areas most affected by this infection.

APPLICATION METHODOLOGY IN SEEDLINGS

In light of the successful results obtained from applying the bioproduct in Petri dishes in laboratory tests with strains of SAPROLEGNIA, an experiment was designed at the Royal Botanical Gardens (CSIC) with cork oak seedlings and pepper plants.

Ten cork oak seedlings (QUERCUS SUBER) about 50 cm long and ten pepper seedlings (CAPSICUM ANNUM) about 30 cm long with no symptoms of infection caused by PHYTOPHTHORA (control) were used. The roots of 5 seedlings of each species were treated with the biocide (liquid and gel) at the minimum inhibitory concentration for 1 h or with distilled water (control) and then exposed to zoospores of P. CINNAMOMI (for cork oak) and P. CAPSICI (for pepper) at concentrations of 10 z/ml for 1 h. After an additional hour, the roots were observed using an inverted microscope.

CONCLUSIONS

Plants treated with the product were not affected by fungal growth on the roots, resulting in 100% prevention of the disease.

Example 3

# EP 4 649 825 A1

APPLICATION TESTS FOR THE PRODUCT BIOCOPAL AGAINST FUNGAL PEST IN CRAYFISH AT THE ROYAL BOTANICAL GARDEN (CSIC). MADRID

## SUMMARY

The product is tested on native crayfish in five aquariums containing ten crayfish each, measuring approximately 10 cm in length. In the infected aquariums, all the crayfish treated with the product survived, while all the untreated crayfish died.

## INTRODUCTION

Fungal pathogens and pseudofungi (such as oomycetes) are responsible for some of the most serious diseases occurring in wildlife. Over the last few decades, natural populations of endangered animal species have experienced an increasing number of fungal infections. Some of the pathogens responsible for these diseases are associated with the first documented extinction events in several species caused by infection, resulting in increasing rates of biodiversity loss (Fisher et al. 2012). Probably one of the most notorious cases is the so-called "crayfish plague" caused by the fungus-like organism APHANOMYCES ASTACI SCHIKORA (oomycetes). This pathogen is considered among the 100 worst invasive species in the world[7] http://www.issg.org, and has destroyed most of the native freshwater crayfish populations in Europe[8] Unestam 1972, Edgerton et al. 2004, Diéguez-Uribeondo et al. 2006.

Over time, the crayfish plague has become one of the most well-known invertebrate disease plagues due to recent advances in developmental biology, cell biology, genomics, molecular taxonomy, and phylogeny. Furthermore, the development of molecular tools for the identification of A. ASTACI is providing a better understanding of the biology and molecular genetics of the crayfish plague pathogen[9] Rezinciuc, R. et al. The Biology of Crayfish Plague Pathogen Aphanomyces astaci. Chapter (2015) Available at:https:Hwww.researchgate.net/publication/280921076 The Biology of Crayfish Plague Pathog en Aphanomyces astaci Current Answers to Most Frequent Questions.

However, research on treatment and control of the disease has not been successful and there is currently a lack of knowledge on the prevention and control of crayfish plague. Preventive treatments against the spread of A. ASTACI are particularly important, as this is a key factor in the rapid mortality of European, Asian and Oceanic species.

In this study, the effect of the product on two species that cause the disease, A. ASTACI, was investigated in: (i) laboratory conditions on different stages of the biological development of these pathogens (related to spread), and (ii) in controlled facilities with crayfish kept in aquariums.

## METHODOLOGY OF APPLICATION IN AQUARIUMS

Five aquariums were used, each containing 10 crayfish of the native Iberian species AUSTROPOTAMOBIUS PALLIPES, approximately 10 cm in length. In the first three aquariums, zoospores of A. ASTACI were added until a concentration of $10^3$ zoospores/ml was achieved. In aquarium number 4, only zoospores were added, and in aquarium 5, no zoospores were added. In each of the aquariums except aquarium 4, a volume of the product was added until the minimum inhibitory concentration according to the *in vitro* experiments was achieved. The product

was added at different times (6, 12, and 24 h in each aquarium) after adding the zoospores to aquariums 1, 2, and 3, respectively.

In aquarium number 4, after exposing the crayfish to zoospores of A. ASTACI, 100% mortality was observed after 10 days. In aquarium number 5, in which the crayfish were only exposed to the treatment, all the crayfish survived after two months. Similarly, in aquariums 1, 2, and 3, none of the specimens were affected by the pathogen and survived for the two months of the experiment.

## CONCLUSIONS

Treatment of crayfish with the product against the disease caused by zoospores of A. ASTACI resulted in the survival of all (100%) individuals compared to untreated individuals, which had a mortality rate of 100%.

## GENERAL CONCLUSIONS

In light of the results of the tests in the laboratory and relevant environment on the formulation of the product, its different presentations, and the effects of each of them on the pathogens SAPROLEGNIA, PHYTOPHTHORA, and APHANOMYCES ASTACI, it can be concluded that:

a) The original formulation of the product described in 3.1 is highly effective as an anti-oomycete.

b) The product has a strong anti-oomycete effect sustained over time in an aqueous medium for at least 48 hours.

c) The product in liquid state that is not synthesised following the methodology described in section 3.1 does not have the OOMYCETE-inhibiting power demonstrated for the original product, and its behaviour in solution is not sustained over time, dissolving immediately.

d) The cured product, in solid state, has no anti-oomycete effect on the growth of fungal cultures in Petri dishes.

e) The cured product, in solid state, dissolves immediately in aqueous solution and provides a concentration of reactants in solution considerably lower than those released by the original product.

f) The product in gel state does not exhibit anti-oomycete activity for ionic gels and exhibits very poor anti-oomycete activity for the product embedded in inert gels, such as potassium polyacrylate.

g) The product in gel state diffuses very little of the gel bead content in aqueous solution, and in the case of dry gel, it absorbs water from the environment.

h) The product is suitable for use as a growth inhibitor of the oomycete PHYTOPHTHORA in trees and plants.

i) The product is suitable for use as a growth inhibitor of the oomycete SAPROLEGNIA in salmonids.

j) The product is suitable for use as a growth inhibitor of the oomycete APHANOMYCES ASTACI in crayfish.

k) Although the experiments described in this specification are very specific to particular real-world environments, this does not limit the scope of use of the product in other animal or plant environments that may be affected by oomycete infestation.

**Claims**

1. A product **characterised by** a production process comprising the following steps:

   a) preparing a 1% acetic acid solution by volume;

   b) heating a given volume of this solution in the range comprised between 50 and 70°C, preferably in a bain-marie;

   c) once heated, adding 1% by weight of chitosan (any deacetylation degree) with respect to the volume of the solution, with constant stirring and maintaining the temperature;

   d) once the chitosan is dissolved, adding 1% by weight of tannic acid with respect to the total volume, in the same solution;

   e) vigorously stirring the mixture, always maintaining the temperature, for the time necessary for complete dissolution (about 1 hour);

   f) once dissolved, being able to add a percentage by weight/volume of not more than 5% of any authorised additive or colouring agent, with the final pH being around 4.

2. A product produced according to the process of claim 1, the technical features of which are:

   a) a liquid product, thicker than water, brown in colour, where the inclusion of tannic acid in the chitosan matrix, stimulated by temperature, slightly alters the polymeric molecular structure of the latter, resulting in a compound that is more insoluble in water than the same product synthesised outside the temperature range specified in claim 1;

   b) a product with a slow diffusion effect in water, with a proven duration of at least 48 hours, which only occurs for the product synthesised by the process specified in claim 1;

   c) a product with a strong, prolonged anti-oomycete effect, being 40% more effective on Petri dish cultures of oomycetes than the product synthesised using another production process and outside the temperature range specified in claim 1; 80% more effective than a product with the same composition in gel form; and 100% more effective than any other product with the same components in solid form.

3. A product obtained according to the process of claim 1, for use in a method for the prevention, treatment and control of the pseudofungus PHYTOPHTHORA in trees and plants affected by the disease, either by endotherapy, by direct irrigation at the roots of the trees, or by any other method of application.

4. A product obtained according to the process of claim 1, for use in a method for the prevention, treatment and control of the pseudofungus SAPROLEGNIA in salmonids by dissolving the product in the in the water used to cultivate fertilised eggs and in the water in tanks containing adult fish.

5. A product obtained according to the process of claim 1, for use in a method for the prevention, treatment and control of the pseudofungus APHANOMYCES ASTACI in crayfish, by means of a method of treatment by dissolving the product in water containing adult specimens or their fertilised eggs.

6. A product obtained according to the process of claim 1, for use in a method for the prevention, treatment and control of anti-oomycetes related to diseases affecting animals and plants.

**Amended claims under Art. 19.1 PCT**

1. A product with anti-oomycete properties to control diseases in silviculture, agriculture, aquaponics and aquaculture, **characterised by** a production process comprising the following steps:

   a) preparing a 1% acetic acid solution by volume;
   b) heating a given volume of this solution in the range comprised between 50 and 70°C, preferably in a bain-marie;
   c) once heated, adding 1% by weight of chitosan (any deacetylation degree) with respect to the volume of the solution, with constant stirring and maintaining the temperature;
   d) once the chitosan is dissolved, adding 1% by weight of tannic acid with respect to the total volume, in the same solution;
   e) vigorously stirring the mixture, always maintaining the temperature, for the time necessary for complete dissolution (about 1 hour);
   f) once dissolved, being able to add a percentage by weight/volume of not more than 5% of any authorised additive or colouring agent, with the final pH being around 4.

2. A product produced according to the process of claim 1, the technical features of which are:

   a) a liquid product, thicker than water, brown in colour, where the inclusion of tannic acid in the chitosan matrix, stimulated by temperature, slightly alters the polymeric molecular structure of the latter, resulting in a compound that is more insoluble in water than the same product synthesised outside the process specified in claim 1;
   b) a product with a slow diffusion effect in water, with a proven duration of at least 48 hours, which only occurs for the product synthesised by the process specified in claim 1.

3. A product obtained according to the process of claim 1, for use in the prevention, treatment and control of infections caused by pseudofungi in silviculture and agriculture.

4. A product obtained according to the process of claim 1, for use in the prevention, treatment and control of infection caused by the pseudofungus Saprolegnia in salmonids.

5. A product obtained according to the process of claim 1, for use in the prevention, treatment and control of infection caused by the pseudofungus Aphanomyces in crayfish.

FIG. 1

FIG. 2

FIG. 3

FIG. 4

FIG. 5

FIG. 6

FIG. 7

FIG. 8

FIG. 9

FIG. 10

FIG. 11

FIG. 12

21

FIG. 13

FIG. 14

FIG. 15

FIG. 16

FIG. 17

FIG. 18

FIG. 19

FIG. 20

FIG. 21

# INTERNATIONAL SEARCH REPORT

| | International application No. |
|---|---|
| | PCT/ES2023/070077 |

## A. CLASSIFICATION OF SUBJECT MATTER

See extra sheet

According to International Patent Classification (IPC) or to both national classification and IPC

## B. FIELDS SEARCHED

Minimum documentation searched (classification system followed by classification symbols)
A01N, A61K, A01P, A61P

Documentation searched other than minimum documentation to the extent that such documents are included in the fields searched

Electronic data base consulted during the international search (name of data base and, where practicable, search terms used)

EPODOC, INVENES, WPI, MEDLINE, BIOSIS,EMBASE, COMPENDEX, INSPEC, NPL, CAPLUS, Internet, BD-TXTE,

## C. DOCUMENTS CONSIDERED TO BE RELEVANT

| Category* | Citation of document, with indication, where appropriate, of the relevant passages | Relevant to claim No. |
|---|---|---|
| X | CN 107586800 A (TAIXING DONGSHENG BIO TECH CO LTD et al.) 16/01/2018, paragraphs 5, 11-27,34, 37; example 7, claims 1, 2-5, 6. | 1-6 |
| A | US 2011059162 A1 (REED JESS DREHER et al.) 10/03/2011, paragraphs 5, 14, 88, 103, 110-113, 116, 127, 151 and 152; examples 5 and 6; figures 3 and 4. | 1-6 |
| A | CN 105079878 A (UNIV JIANGNAN) 25/11/2015, example 1, step 1. | 1-6 |
| A | RUBENTHEREN V et al. Processing and analysis of chitosan nanocomposites reinforced with chitin whiskers and tannic acid as a crosslinker. Carbohydrate Polymers England, 2015, vol. 115, pages 379 - 387, ISSN 1879-1344 (Electronic), DOI: 10.1016/j.carbpol.2014.09.007pubmed:25439908, paragraphs 2.3 and 2.4, table1, samples II A and B | 1-6 |

☒ Further documents are listed in the continuation of Box C.     ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other documents , such combination being obvious to a person skilled in the art |
| "O" document referring to an oral disclosure use, exhibition, or other means. | |
| "P" document published prior to the international filing date but later than the priority date claimed | "&" document member of the same patent family |

| Date of the actual completion of the international search | Date of mailing of the international search report |
|---|---|
| 28/09/2023 | **(29/09/2023)** |

| Name and mailing address of the ISA/ | Authorized officer |
|---|---|
| OFICINA ESPAÑOLA DE PATENTES Y MARCAS Paseo de la Castellana, 75 - 28071 Madrid (España) Facsimile No.: 91 349 53 04 | A. Polo Diez |
| | Telephone No. 913495524 |

Form PCT/ISA/210 (second sheet) (July 2022)

## INTERNATIONAL SEARCH REPORT

International application No.

PCT/ES2023/070077

**C (continuation).** DOCUMENTS CONSIDERED TO BE RELEVANT

| Category * | Citation of documents, with indication, where appropriate, of the relevant passages | Relevant to claim No. |
|---|---|---|
| A | RIVERO S et al. Heat treatment to modify the structural and physical properties of chitosan-based films. Journal Of Agricultural And Food Chemistry, 2012, vol. 60 (1), pages 492 - 499, ISSN 0021-8561 (print) ISSN 1520-5118 (electronic), DOI:10.1021/jf204077k> | 1 |
| A | EP 3318125 A1 (CHEMIA S P A) 09/05/2018, Example 3 | 1 |
| A | HUANG X et al. Antifungal activity of chitosan against *Phytophthora infestans*, the pathogen of potato late blight. International Journal Of Biological Macromolecules, 2021, vol. 166, pages 1365- 1376, ISSN 0141-8130 (print) ISSN 1879-0003 (electronic), DOI: 10.1016/j.ijbiomac.2020.11.016pubmed:33161079 | 1-6 |
| A | MIN HONG-KYU et al. Some inhibitory effects of chitosan on fish-pathogenic oomycete, *Saprolegnia parasitica*. Fish Pathology, 1994, vol. 29 (2), pages 73-77, ISSN 0388-788X | 1-6 |
| A | AHMED FATMA et al. In vitro assessment of the antimicrobial efficacy of chitosan nanoparticles against major fish pathogens and their cytotoxicity to fish cell lines. Journal Of Fish Diseases, 2020, vol. 43 (9)9, pages 1049-1063, ISSN 1365-2761 (Electronic), DOI: 10.1111/jfd.13212 pubmed:32632933 | 1-6 |
| A | ZHANG Q Q et al. Inhibitory Effects of Galla chinensis, Tannic Acid, and Gallic Acid on *Saprolegnia parasitica*. Israeli Journal Of Aquaculture Bamidgeh, 2019, vol. 71, Article N°: 1629, ISSN 0792-156X | 1-6 |

Form PCT/ISA/210 (continuation of second sheet) (July 2022)

## INTERNATIONAL SEARCH REPORT

Information on patent family members

International application No.

PCT/ES2023/070077

| Patent document cited in the search report | Publication date | Patent family member(s) | Publication date |
|---|---|---|---|
| CN107586800 A | 16.01.2018 | NONE | |
| US2011059162 A1 | 10.03.2011 | US2017142967 A1<br>US10104888 B2<br>US2014134238 A1<br>US9545423 B2<br>US8642088 B2 | 25.05.2017<br>23.10.2018<br>15.05.2014<br>17.01.2017<br>04.02.2014 |
| CN105079878 A | 25.11.2015 | NONE | |
| EP3318125 A1 | 09.05.2018 | ES2748901T T3<br>PL3318125T T3<br>PT3318125T T<br>IT201600111393 A1 | 18.03.2020<br>31.01.2020<br>25.10.2019<br>04.05.2018 |

Form PCT/ISA/210 (patent family annex) (July 2022)

## INTERNATIONAL SEARCH REPORT

| International application No. |
| --- |
| PCT/ES2023/070077 |

CLASSIFICATION OF SUBJECT MATTER

*A01N43/16* (2006.01)
*A01N31/08* (2006.01)
*A61K31/722* (2006.01)
*A61K31/192* (2006.01)
*A01P1/00* (2006.01)
*A61P31/00* (2006.01)

Form PCT/ISA/210 (extra sheet) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 105079878 A
- US 2011059162 A1
- EP 3318125 A1
- ES 2761076 A1

**Non-patent literature cited in the description**

- **RAFAAT, DINA et al.** *Microbial Biotechnology*, 2009, vol. 2, 186-201
- **ZHENG, K. et al.** *International Journal of Biological Macromolecules*, 2021, vol. 182, 1670-1680
- **STUART, B.H.** *Vibrational Spectroscopy*, 1996, vol. 10, 79-87
- **ZAJAC, A. et al.** *Spectrochimica Acta Part A: Molecular and Biomolecular Spectroscopy*, 2015, vol. 134, 114-120
- **SANDOVAL-SIERRA, J.V. et al.** *Aquaculture*, 2014, vol. 434, 462-469
- **HENDRICK VAN DEN BERG, A. et al.** *Fungal Biology Reviews*, 2013, vol. 27 (2), 33-42
- **REZINCIUC, R. et al.** The Biology of Crayfish Plague Pathogen Aphanomyces astaci.. 2015